**Europäisches Patentamt**

**European Patent Office**

**Office européen des brevets**

(19)

(11) Veröffentlichungsnummer: **0 008 647 B1**

(12)

# EUROPÄISCHE PATENTSCHRIFT

(45) Veröffentlichungstag der Patentschrift:
**30.12.81**

(21) Anmeldenummer: **79102510.9**

(22) Anmeldetag: **18.07.79**

(51) Int. Cl.³: **A 61 F 9/00**

(54) **Vorrichtung zur Wiederanlegung einer abgelösten Netzhaut des Auges.**

(30) Priorität: **22.07.78 DE 2832312**

(43) Veröffentlichungstag der Anmeldung:
**19.03.80 Patentblatt 80/6**

(45) Bekanntmachung des Hinweises auf die Patenterteilung:
**30.12.81 Patentblatt 81/52**

(84) Benannte Vertragsstaaten:
**CH FR GB NL**

(56) Entgegenhaltungen:
**DE-A1-2 351 247**
**DE-A-2 363 192**
**DE-B2-2 722 252**
**DE-B1-2 832 312**
**GB-A-1 121 710**
**US-A-3 528 410**

**TRANSACTIONS OF THE AMERICAN
ACADEMY OF OPHTHALMOLOGY AND
OTOLARYNGOLOGY
Band 73, November/Dezember 1969, Rochester
D.B. KARLIN: "Ultrasound in retinal detachment
surgery"
Seiten 1061 bis 1076**

(73) Patentinhaber: **DORNIER SYSTEM GmbH, Postfach 1360,
D-7990 Friedrichshafen (DE)**

(72) Erfinder: **Forssmann, Bernd, Dr., Manzeller Strasse 18/1,
D-7990 Friedrichshafen 1 (DE)**
Erfinder: **Hepp, Wolfgang, Dr., Hersbergweg 16,
D-7759 Immenstaad am Bodensee (DE)**
Erfinder: **Trier, Hans-Georg, Dr., Höhenweg 1,
D-5300 Bonn (DE)**

(74) Vertreter: **Landsmann, Ralf, Dipl.-Ing., Kleeweg 3,
D-7990 Friedrichshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Vorrichtung zur Wiederanlegung einer abgelösten Netzhaut des Auges

Die Erfindung betrifft eine Vorrichtung zur Wiederanlegung einer abgelösten Netzhaut des Auges an das Pigmentepithel und die darunterliegende Aderhaut mittels Stosswelle.

Die Netzhaut (Retina) liegt im Normalzustand auf ihrer gesamten Umfangsfläche auf dem Pigmentepithel lose auf, das seinerseits der darunterliegenden Aderhaut anhaftet. Nur an dem etwa äquatorial verlaufenden Rand der Netzhaut (Ora serrata) und am Sehnerv ist die Netzhaut fester mit der Unterlage verbunden. Im übrigen Verlauf werden beide Häute durch den Quellendruck des Glaskörpers in Kontakt und auf der geometrisch definierten Innenfläche des Augapfels gehalten.

Die loch- oder rissbedingte Netzhautablösung

Gewalteinwirkung auf das Auge (Trauma) sowie krankhafte Vorgänge im Glaskörper (Schrumpfung, Zug an der Netzhaut) können zu Einrissen der Netzhaut führen; degenerative Veränderungen in der Netzhaut selbst können in ihr Löcher verursachen. Wenn der Glaskörper in der Umgebung seine normale Struktur verloren und sich verflüssigt hat, und der verflüssigte Glaskörper durch den Riss oder das Loch der Netzhaut zwischen diese und das Pigmentepithel eindringt, entsteht eine fortschreitende Vorwölbung der Netzhaut, die sogenannte Netzhautablösung, wodurch in den betroffenen Bezirken die Sehfunktion weitgehend erlischt.

Durch den Riss oder das Loch in der abgelösten Netzhaut steht das Volumen des Glaskörpers mit dem durch die Ablösung gebildeten Spaltraum zwischen Netzhaut und Pigmentepithel in Verbindung.

Die nicht lochbedingte Netzhautablösung

Durch Austritt von Flüssigkeit aus der Aderhaut und ihr Eindringen zwischen Netzhaut und Pigmentepithel kann es auch ohne Vorliegen eines Nezthautlochs oder -risses zu einer Netzhautablösung kommen.

Das Prinzip der Behandlung der loch- oder rissbedingten Netzhautablösung besteht in der Abdichtung des Netzhautloches bzw. -risses gegen den Glaskörper. Hierzu muss die abgelöste Netzhaut in der Umgebung des Lochs bzw. des Risses an das Pigmentepithel mit der Aderhaut zunächst wieder angelegt werden und dann mit ihr durch Narbenbildung dicht verbunden werden. Für die oft schwierige Wiederanlegung werden verschiedene Verfahren eingesetzt, unter anderem die Lagerung des Patienten, die operative Punktion der subretinalen Flüssigkeit und häufig die eindellende Operation, bei der die Augenwand durch mehr oder weniger starkes Eindellen der abgehobenen Netzhaut genähert oder angelegt wird. Diese operativen Therapiemethoden, besonders die eindellende Operation, stellen jedoch äusserst komplizierte, für den Patienten stark belastende und nicht selten vergebliche Eingriffe dar.

Als nicht invasive Methode bei loch- oder rissbedingter Ablösung sind bisher zur Wiederanlegung der Netzhaut Versuche mit Ultraschall durchgeführt worden (u. a. Purnell, E. W.: Therapeutic use of ultrasound. In: Ultrasonics in Ophthalmology, diagnostic and therapeutic applications, Goldberg, R. and Sarin, L., ed., Saunders Co., Philadelphia 1967, 102 – 123; Karlin, D. B.: Ultrasound in retinal detachment surgery, Trans. amer. Acad. Ophthal. Otolarnyng. 73, 1969, 1061-1076), die nur in günstigen Fällen zum Erfolg geführt haben. Dies war dann der Fall, wenn die Rückstellkraft der Netzhaut infolge Adhäsionen mit dem Glaskörper nicht ausgeprägt und der Schallwellenwiderstandsunterschied vor und hinter der abgelösten Netzhaut gross genug war. Mit gewebsverträglichen Schalleistungen liess sich in vielen Fällen die Netzhaut infolge des zu geringen Schallstrahlungsdruckes nicht bewegen.

GB-A- 1 121 710 zeigt und beschreibt ein mechanisches Vibrationsgerät, das durch Berührungskontakt mit der Hornhaut des Auges Vibrationen in das Auge einleitet. Mit diesem Gerät soll die Netzhaut in ihre Ausgangslage zurückgetrieben werden. Dabei wird das Auge an einem Breitenkreis in Schwingungen versetzt. Es ist unwahrscheinlich und physikalisch wie auch medizintechnisch nicht zu erwarten, dass sich durch Vibrationen eine Kugelwellenfront ausbildet. Durch die hin- und hergehende Bewegung eines Stempels wird selbst im idealisierten Fall eine toroidförmige Wellenfront erzeugt, die durch unterschiedlich starken Berührungskontakt des Stempels mit dem Auge einen völlig unkontrollierten Verlauf nimmt, so dass die Gefahr einer Schädigung des Auges besteht.

DE-A1- 23 51 247 ist ein Gerät zur Erzeugung von Stosswellen.

Mit einer fokussierten Stosswelle gemäss dieser Anmeldung werden Abplatzeffekte an Konkrementen bewirkt, d.h. es werden Zerstörungen dort verursacht, wo ein unterschiedlicher Schallwellenwiderstand vorliegt. Nun darf man jedoch die Netzhaut des Auges keinesfalls zerstören, so dass die Lehre dieser DE-OS nicht auf das Auge übertragbar ist.

Der Erfindung liegt die Aufgabe zugrunde, eine Vorrichtung zu schaffen, die auf möglichst kontrollierte Weise die berührungsfreie Wiederanlegung einer abgelösten Netzhaut des Auges an das Pigmentepithel und die darunterliegende Aderhaut ermöglicht.

Diese Aufgabe wird erfindungsgemäss dadurch gelöst, das ein Stosswellengenerator mit einer in einem Reflektor justierbaren Funkenstrecke vorgesehen ist, die eine Stosswellenfront ausendet, dass der Innenraum des Reflektors mit einer Flüssigkeit gefüllt ist und dass der Reflektor gegenüber dem Auge mit einer elastischen Membran verschlossen ist und die Membran am Auge anliegend eine formangepasste Stoswellenfront in das Auge einleitet, wo sie die Netzhaut langsam in ihre Ausgangslage zurücktreibt.

Während Ultraschallwellen in gewöhnlicher Weise einen Nulldurchgang im Druck-Zeit-Diagramm aufweisen und somit eine Druck- und Zugwechselwirkung an einem bestrahlten Körper verursachen, weisen Druckimpulse oder Stosswellen in Flüssigkeiten keinen Nulldurchgang auf und sind folglich als kurzzeitige Kraftstösse in der Lage, einen Körper zu verschieben.

Die Erfindung bietet folgende Vorteile:
- Es wird ein kurzzeitiger flächenhafter Kraftstoss auf die Netzhaut ausgeübt, der geeignet ist, Verklebungen der Netzhaut am Glaskörper zu lösen oder um Netzhautfalten zu lösen.
- Der kurzzeitige Kraftstoss ist intensiv genug, um die hinter der Netzhaut befindliche Flüssigkeit durch Perforationen in der Netzhaut nach vorn auszutreiben; oder um bei gleichzeitiger Punktion den Abfluss dieser Flüssigkeit nach aussen zu erleichtern.
- Aufgrund von Laufzeiteffekten kann die resultierende Stossfront so geformt oder fokussiert werden, dass die Netzhaut von aussen beginnend zum Ort der Perforation in der Netzhaut oder zum Ort einer Punktion hin langsam fortschreitend angelegt wird.

Weitere Vorteile, Merkmale und Anwendungsmöglichkeiten ergeben sich aus den Figuren, die nachfolgend beschrieben sind.

Es zeigt
Fig. 1 ein Auge mit abgelöster Netzhaut in Schnittdarstellung,
Fig. 2, 3, 4 und 5 die prinzipielle Darstellung der Wiederanlegung der Netzhaut mittels Stosswellen und
Fig. 6 ein Gerät zur Erzeugung der hier verwendeten Art in Schnittdarstellung.

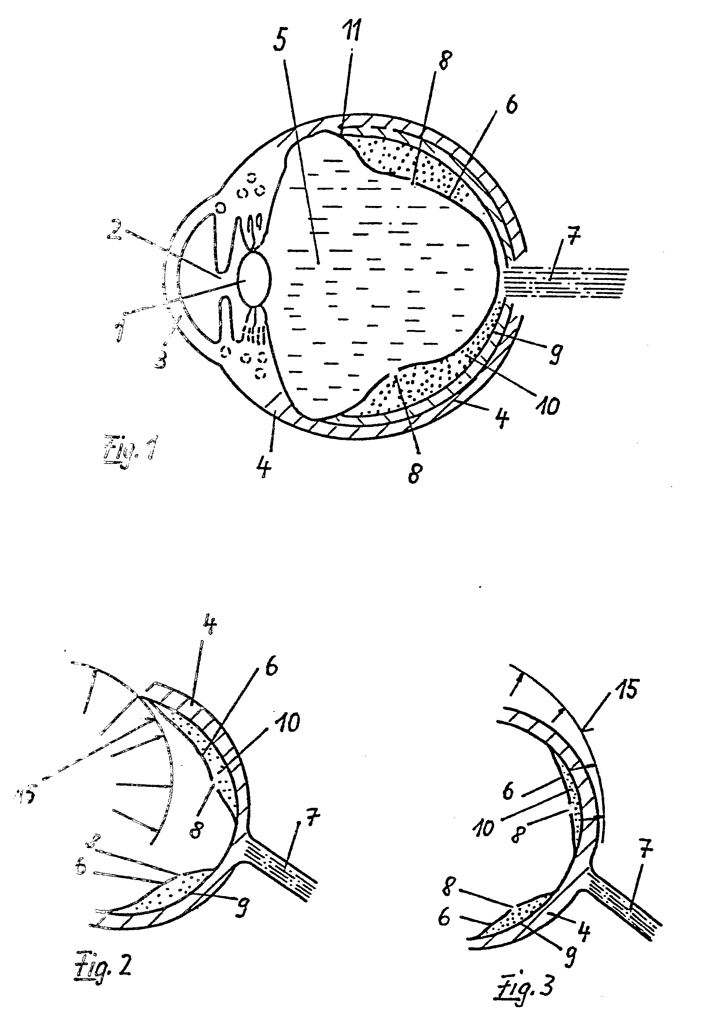

Fig. 1 zeigt die Schnittdarstellung durch ein Auge, wobei die wichtigsten Bestandteile folgende Bezugsziffern erhalten haben:

| | |
|---|---|
| 1 Augenlinse | 8 Verletzungen der Netzhaut |
| 2 Pupille/Iris | (Riss, Loch) |
| 3 Hornhaut | 9 Aderhaut und |
| 4 Lederhaut | Pigmentepithel |
| 5 Glaskörper | 10 Flüssigkeit zwischen |
| 6 Netzhaut | Aderhaut oder Pigmentepithel |
| 7 Sehnerv | und abgelöster Netzhaut |
| | 11 Ora serrata |

Im rechten Teil von Fig. 1 ist deutlich die Netzhaut 6, die Aderhaut mit Pigmentepithel 9 und die Lederhaut 4 zu erkennen. Die Netzhaut liegt beim gesunden Auge flächenhaft an der Aderhaut mit Pigmentepithel 9 an. Beim hier krankhaft veränderten Auge hat sich die Netzhaut in weiten Bereichen von der Aderhaut mit Pigmentepithel 9 abgelöst. Sie ist lediglich noch bei 11 an der Lederhaut 4 befestigt.

Zwischen Netzhaut und Pigmentepithel hat sich ein Spalt 10 gebildet, der mit einer interstitiellen Flüssigkeit gefüllt ist. In der Zeichnung sind deutlich Einrisse und Perforationen 8 in der Netzhaut zu erkennen. Wegen der Ablösung der Netzhaut droht in diesen Bereichen die Sehfunktion zu erlöschen.

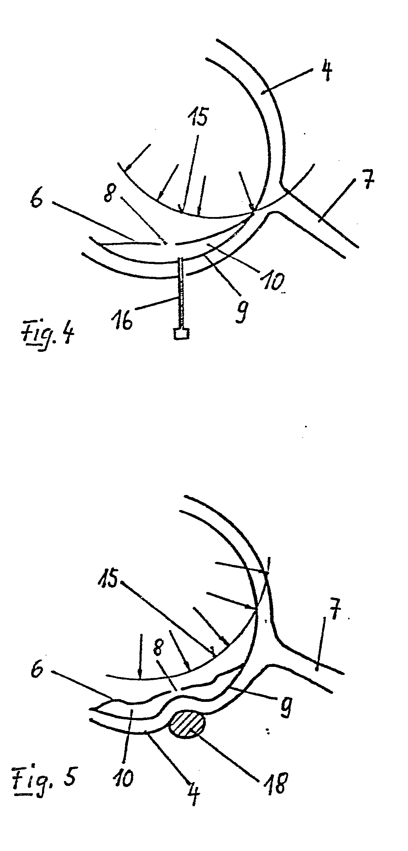

Fig. 2 und 3 zeigen nun in prinzipieller Darstellung, wie es gelingt, mit Hilfe von Stosswellen die Netzhaut 6 erneut an die Aderhaut 9 flächenhaft anzulegen. Gelangt eine Stossfront 15, die als geschlossener Kurvenzug gezeichnet ist, in den Bereich der Netzhaut 6, so wird durch sie eine Verschiebewirkung ausgelöst, die die Netzhaut an die Aderhaut mit Pigmentepithel anzulegen vermag. Die Flüssigkeit, die sich im Spalt 10 befindet, kann diesen durch die Perforation 8 verlassen. Wie aus Fig. 2 und 3 deutlich hervorgeht, ist die Stosswellenfront 15 der Form der Netzhaut, bzw. der dahinterliegenden Lederhaut angepasst, so dass eine flächenhafte Anlegung erfolgen kann. Mit der hier gezeigten Stossfront wird die Netzhaut im oberen Teil des Bildes angelegt, während zur Anlegung der im unteren Teil des Bildes vorhandenen abgelösten Netzhaut es erforderlich ist, eine Stosswellenfront anderer Geometrie zu erzeugen.

Im einzelnen wird beispielsweise folgendermassen vorgegangen:

Eine z. B. kugelförmige Stosswelle wird so erzeugt, dass sie dort zuerst auf die Netzhaut 6 trifft, wo diese noch mit der Lederhaut 4 verbunden ist. Der im Spalt 10 befindliche Flüssigkeitsteil wird zur Mitte der Netzhaut 6 beschleunigt und treibt einen Teil der hinter der Netzhaut befindlichen Flüssigkeit durch die Perforation 8 in der Netzhaut hindurch nach vorn. Geht man davon aus, dass die Einleitung der Stosswellen 15 unter Sicht erfolgt, so kann durch geeignete Positionierung der Stosswellenquellen erreicht werden, dass diese günstige Geometrie auch bei den folgenden Stössen beibehalten wird und damit die in den Randbezirken befindlichen Flüssigkeitsmengen zur Mitte hin beschleunigt werden.

Falls die Austreibung der Flüssigkeit unter der Netzhaut nach vorne durch Perforationen in der Netzhaut nicht oder nicht ausreichend durchführbar ist oder wegen Fehlens von Perforationen ausscheidet (in Fällen von Ablösung infolge Austritts von Flüssigkeit aus der Aderhaut zwischen Netzhaut und Pigmentepithel), kann eine Punktionskanüle 16 (Fig. 4) in den Spalt 10 eingeführt werden, durch die die Flüssigkeit nach aussen abfliessen kann. Der Abfluss kann durch geeignete Anpassung der Stosswellenfront 15 erleichtert oder vervollständigt werden.

Die geschilderte Anlegung durch Stosswellen kann einerseits anstelle einer Eindellung 18 (Fig. 5) durchgeführt werden, andererseits auch während oder nach einer eindellenden Operation erfolgen, um die Anlegung der Netzhaut an die eingedellte Aderhaut zu beschleunigen oder zu vervollständigen.

Wurde die Netzhaut 6 in ihre ursprüngliche Lage zurückgebracht, so muss sie fixiert werden. Dies kann in der Weise erfolgen, dass durch eine Licht-, Laser-, Ultraschallkoagulation-, Kälte- oder Diathermiebehandlung die Netzhaut 6 mit dem Pigmentepithel und der Aderhaut 9 verschweisst wird. Eine Fixierung der Netzhaut kann auch so erreicht werden, dass bis zum natürlichen Anwachsen die Netzhaut mit Ultraschall oder sehr schwachen Stosswellen in der angelegten Lage festgehalten wird.

Bei Versuchen im Modell an Kunststoffolien hat sich gezeigt, dass Stosswellen im Bereich von 70 bar zur Verschiebung der Netzhaut geeignet erscheinen. Bei Stosswellen dieser Grössenordnung ist auch nach heutigem Wissen nicht zu erwarten, dass Gewebeschäden oder Funktionsstörungen durch die Behandlung auftreten könen. Für die Netzhautanlegung wird ein Druckbereich gewählt, in dem die Anlegung langsam und kontrollierbar erfolgt.

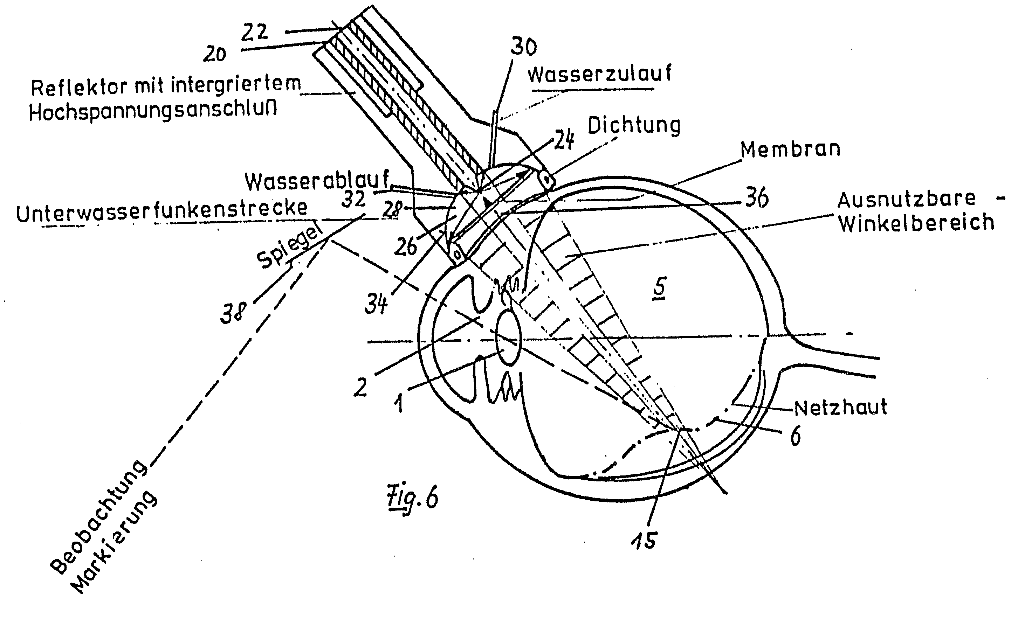

Fig. 6 zeigt die Schnittdarstellung eines Gerätes zur Erzeugung von Stosswellen, das erfindungsgemäss zur Wiederanlegung der Netzhaut verwendet wird.

Für die Erzeugung der Stosswellen eignet sich bei klinischer Anwendung die Zündung eines Unterwasserfunkens. Hierzu wird ein Hochspannungsimpuls über die Anschlüsse 20 und 22 auf die Unterwasserfunkenstrecke 24, die hier prinzipiell durch zwei Pfeile dargestellt wird, geleitet. Die Funkenstrecke 24 selbst befindet sich in einer Kammer 26, die mit einer Flüssigkeit 28, z. B. Wasser, gefüllt ist. Die Kammer 26 wird durch einen Zufluss 30 und einen Abfluss 32 ständig gespült, um Gasblasen, die bei der Zündung des Funkens entstehen, abzuführen. Die Funkenstrecke 24 kann als freie Funkenstrecke im Innern der Kammer 26 oder als Gleitfunkenstrecke auf der Kammerinnenwand 34 ausgeführt sein. Die Kammer ist durch eine elastische verformbare Membran 36 abgeschlossen, die sich im Anwendungsfall direkt an das Auge anschliesst. Mit Hilfe eines Spiegels 38 kann der Anlegungsvorgang beobachtet werden.

Die gewünschte Form der Stosswellenfront für die Anlegung der Netzhaut wird durch die Geometrie der Innenwand 34 und der Funkenstrecke 24 bestimmt (z. B. eine ebene Stosswellenfront durch einen parabolischen Reflektor mit freier Funkenstrecke im Brennpunkt) und durch die Zündfolge, wenn mehrere Stosswellenimpulse hintereinander ausgelöst werden und sich additiv überlagern. Die Funkenstrecke 24 kann im Reflektor verschoben werden und durch ihre neue Lage ergibt sich eine veränderte Stosswellenfront 15.

In einer weiteren Anwendung kann die einzelne Funkenstrecke durch eine Mehrfachfunkenstrecke ausgetauscht werden (nicht gezeigt). Mit einer solchen Funkenstrecke lassen sich Stossfronten in jeder gewünschten Form erzeugen, da sich die Stossfronten der einzelnen Impulse immer additiv überlagern. Eine weitere Möglichkeit, die gewünschte Form der Stosswellenfront zu erhalten, besteht darin, dass die einzelnen Funken der Mehrfachfunkenstrecke in gleicher zeitlicher Reihenfolge aufeinanderfolgend gezündet werden. Für eine derartige Anwendung wird zweckmässigerweise die Funkenstrecke in Fig. 6 als Gleitfunkenstrecke ausgebildet.

## Patentansprüche:

1. Vorrichtung zur Wiederanlegung einer abgelösten Netzhaut des Auges an das Pigmentepithel und die darunterliegende Aderhaut mittels Stosswelle, dadurch gekennzeichnet, dass ein Stosswellengenerator mit einer in einem Reflektor justierbaren Funkenstrecke (24) vorgesehen ist, die eine Stosswellenfront aussendet, dass der Innenraum des Reflektors mit einer Flüssigkeit (28) gefüllt ist und dass der Reflektor gegenüber dem Auge mit einer elastischen Membran (36) verschlossen ist und die Membran (36) am Auge anliegend eine formangepasste Stosswellenfront (15) in das Auge einleitet, wo sie die Netznaut (6) langsam in ihre Ausgangslage zurücktreibt.

2. Vorrichtung nach Anspruch 1, dadurch gekennzeichnet, dass mehrere Stosswellenfronten (15) aufeinenderfolgend zündbar sind.

3. Vorrichtung nach Anspruch 2, dadurch gekennzeichnet, dass mehrere Stosswellenfronten (15) in gleicher zeitlicher Reihenfolge aufeinanderfolgend zündbar sind.

4. Vorrichtung nach Anspruch 1, dadurch gekennzeichnet, dass mehrere Funkenstrecken (24) unterschiedlicher Geometrie im Reflektor angeordnet sind.

5. Vorrichtung nach einem der Ansprüche 1 bis 4, dadurch gekennzeichnet, dass eine Optik zur Beobachtung des Anlegevorgangs der Netzhaut integriert vorhanden ist oder dank der Formgebung der Vorrichtung simultan mit ihrem Betrieb verwendet werden kann.

## Claims

1. A device for reattaching a detached eye retina to the pigmented epithelium and the choroid membrane beneath it by means of shock waves, comprising a shock wave generator with a spark gab (24) adjustable in a reflector, the spark gap generating a shock wave front, the interior of the reflector being filled with a fluid (28) and the reflector being sealed towards the eye by en elastic membrane (36), the membrane (36) contacting the eye and introducing a shape-fitting shock-wave front (15) into the eye which drives the retina (6) slowly back to its initial position.

2. A device according to claim 1 in which said spark gap is adapted to generate several shock-wave fronts (15) consecutively.

3. A device according to claims 1 and 2 in which said spark gap is adapted to generate several shock-wave fronts (15) in the same time sequence consecutively.

4. A device according to claim 1 including several spark gaps (24) of different geometries mounted in the reflector.

5. A device according to any of the claims 1 to 4 in which an optic means for observing the re-laying procedure of the retina is integrated or the device being shaped so that the operation of the optic means is allowed simultaneously to its operation.

**Revendications**

1. Appareil pour rattacher une rétine détaché de l'œil, à l'épithélium pigmentaire et à la choroïde sous-jacente, au moyen d'ondes de choc, caractérisé en ce qu'il est prévu un générateur d'ondes de choc comportant un éclateur (24) ajustable dans un réflecteur, et émettant un front d'ondes de choc, en ce que l'espace intérieur du réflecteur est rempli d'un liquide (28) et en ce que le réflecteur est fermé envers l'œil par une mem-brane élastique (36) et la membrane (36) étant ap-pliquée sur l'œil engendre dans l'œil un front d'ondes de choc (15) en forme adaptée qui ramène lentement la rétine (6) à sa position initiale.

2. Appareil selon la revendication 1, caractérisé en ce que plusieurs fronts d'ondes dechoc (15) peuvent être amorcés en succession.

3. Appareil selon la revendication 2, caractérisé en ce que plusieurs fronts d'ondes de choc (15) peuvent être amorcés en succession dans le même ordre chronolgique.

4. Appareil selon la revendication 1, caractérisé en ce que plusieurs éclateurs (24) de différentes gométries sont disposés dans le refléc-teur.

5. Appareil selon l'une des revendications 1 à 4, caractérisé en ce qu'un instrument optique est intégré à l'appareil pour l'observation du processus de rattachement de la rétine ou, grâce à la forme donnée à cet appareil, peut être utilisé simultanément avec ce dernier au cours de son fonctionnement.

*Fig. 1*

*Fig. 2*

*Fig. 3*

Fig. 4

Fig. 5

22
20
30
Wasserzulauf
Reflektor mit intergriertem
Hochspannungsanschluß
Dichtung
24
Membran
Wasserablauf
36
Ausnutzbare –
Winkelbereich
Unterwasserfunkenstrecke
32
28
Spiegel
26
5
38
34
2
1
Netzhaut
6
Beobachtung
Markierung
Fig. 6
15

0 008 647